# EUROPEAN PATENT SPECIFICATION

(11) **EP 1 787 651 B1**
(45) Date of publication and mention of the grant of the patent: **10.02.2010**
(21) Application number: 06024137.9
(22) Date of filing: 21.11.2006
(51) Int. Cl.: A61K 35/74, A23C 9/127, A23F 3/16, A23C 9/123

(54) **Bacterial culture having effects of suppressing dermatitis development and accelarating skin wound healing, and product using the same**
Bakterienkultur die Dermatitis hemmt und die Heilung von Hautwunden beschleunigt, und deren Verwendung
Culture bactérielle qui supprime la dermatite et accélére la guérison des lesions cutanées, et son utilisation

(30) Priority: 22.11.2005 JP 2005337640
(43) Date of publication of application: 23.05.2007
(73) Proprietor: Carrozzeria Japan Co., Ltd., Chuo-ku Tokyo (JP)
(72) Inventor: Takeda, Kazunori, Hakusan-shi, Ishikawa-ken (JP)
(74) Representative: Glawe, Delfs, Moll

(56) References cited:
- EP-A- 1 607 476
- DE-A1-102004 051 717
- JP-A- 11 276 072
- JP-A- 2004 149 442
- JP-A- 2005 013 211
- JP-A- 2006 271 219
- JP-A- 2006 271 220
- US-B1- 6 383 482
- ANGELES C B ET AL: "A randomized controlled clinical trial on the efficacy of green tea extract in reducing UVB-induced erythema at 2xMED." JOURNAL OF INVESTIGATIVE DERMATOLOGY, vol. 121, no. 1, July 2003 (2003-07), page 1258, XP009076204 & INTERNATIONAL INVESTIGATIVE DERMATOLOGY 2003 : JOINT MEETING OF THE EUROPEAN SOCIETY FOR DERMATOLOGI; MIAMI BEACH, FLORIDA, USA; APRIL 30-MAY 04, 2003 ISSN: 0022-202X
- VALDÉZ J.C. ET AL: 'Interference of Lactobacillus plantarum with Pseudomonas aeruginosa in vitro and in infected burns: the potential use of probiotics in wound treatment.' CLINICAL MICROBIOLOGY AND INFECTION vol. 11, no. 6, June 2005, pages 472 - 479, XP002511012
- WESTON S. ET AL: 'Effects of probiotics on atopic dermatitis: a randomised controlled trial.' ARCHIVES OF DISEASE IN CHILDHOOD vol. 90, no. 9, 29 April 2005, pages 892 - 897, XP009066050
- CHAPAT LUDIVINE ET AL: 'Lactobacillus casei reduces CD8+ T cell-mediated skin inflammation.' EUR. J. IMMUNOL. vol. 34, no. 9, September 2004, pages 2520 - 2528

## Description

### [Technical Field]

The present invention relates to bacterial cultures having effects of suppressing dermatitis development and accelerating skin wound healing in human or animals, which can be used as raw materials for topical drugs (ointments), skin disorder drugs, cosmetics, drugs such as orally administered drugs, and quasi-drugs for prevention or improvement or can be used by being added to these products or their raw materials. More specifically, the present invention relates to cultures prepared by culturing bacteria in the presence of green tea. Furthermore, the present invention relates to topical drugs (ointments), skin disorder drugs, cosmetics, drugs such as orally administered drugs, and quasi-drugs for prevention or improvement that use the bacterial cultures.

### [Background Art]

Atopic dermatitis, a typical dermatitis, is a disease characterized by eczema with itching caused by skin inflammation due to an allergic reaction of allergens such as tick, house dusts, pollen, mold, and some foods, or caused by dryness of the skin (dry skin) and defensive dysfunction.

As traditional therapies, generally, a long-term therapy by improving the living environment (diet cure and elimination of environmental allergens) has been performed. In addition, as a method for suppressing inflammation, which is a symptom, a steroid topical drug, Tacrolimus hydrate, or a non-steroid topical drug is, generally, directly applied to the inflammation site. Furthermore, as a method for suppressing itching, an anti-histamine drug is orally administered.

On the other hand, skin wounds are a damage of skin surface tissues caused by lacerations, bums, abraded wounds, surgeries, or injuries. Skin wounds are generally cured by recuperative powers and natural healing powers of living bodies after a first-aid treatment.

However, the recovery by natural healing powers takes a long time. In addition, there are influences of continuous pain and bacterial infection. Therefore, therapies for improving recuperative power, reducing pain, preventing bacterial infection, and decreasing inflammation have been performed by directly applying therapeutic drugs to wound sites.

As skin disorder drugs used for curing skin disorders such as dermatitis and skin wounds, steroid topical drugs such as diflorasone acetate, betamethasone valerate, flucinonolone acetonide, dexamethasone, fluocinonide, and alclometasone propionate; non-steroid topical drugs such as bufexamac, bendazac, and sprofen; atopic dermatitis drugs such as Tacrolimus hydrate; and anti-ulcer drugs for skin such as Elase, lysozyme chloride, calf blood extract, and tretinoin tocoferil have been used.

It is reported that strong steroid topical drugs cause side effects such as fever and chronically developing osteoporosis and arteriosclerosis by a long-term administration to a wide area of a subject's body. In addition, it is suggested that administration of a large amount of a strong steroid topical drug to an infant or a child for a long term may suppress an increase in the body height and cause growth depression. Furthermore, steroid topical drugs are not applicable to skin infection by bacteria and fungi. Antiinflammatory analgesic topical drugs of non-steroid topical drugs have an antiinflammatory effect lower than those of steroid topical drugs and are used mainly for mild cases. Almost no side effects are observed, but skin rash and irritation are strongly sensed. Tacrolimus hydrate, which is an immunosuppressive drug of a non-steroid topical drug and is used as an atopic dermatitis drug, has skin irritation. In addition, the carcinogenicity is reported outside Japan. Furthermore, since Tacrolimus hydrate has been launched recently and the safety has not been confirmed yet, it is determined not to apply Tacrolimus hydrate to newborn infants, infants, babies, and children. In addition, the application to a pregnant woman and woman having possibility of being pregnant is forbidden.

Elase, lysozyme chloride, calf blood extract, and tretinoin tocoferil, which are used as anti-ulcer drugs for skin, are forbidden from being applied to subjects who sensitively react on each individual drug. Furthermore, symptoms such as shock, irritation of skin, and rubefaction may be caused as side effects depending on the drugs.

### [Prior Art]

Conventionally, there have been many cautionary points regarding side effects and use of skin disorder drugs having effects of suppressing dermatitis development and accelerating skin wound healing in human or animals. Hence, the development of highly safe skin disorder drugs that have high effects of suppressing dermatitis development and accelerating skin wound healing, have high versatility, and are derived from natural substances has been desired.

As skin disorder drugs utilizing lactic acid bacteria as a natural substance and relating to suppression of dermatitis development, an anti-allergy composition prepared by suspending lymphocytes derived from mouse spleen sensitized with lactic acid bacteria and egg albumin in a medium containing egg albumin (JP-a-2005-139160); a drink or a food containing lactic acid bacteria and a variant thereof having a high anti-allergy activity as an effective ingredient and having an anti-allergy function (JP-A-2005-137357); and a food utilizing resident lactic acid bacteria in the intestines of human and having an anti-allergy effect for improving and preventing allergic diseases such as hay fever, allergic bronchial asthma, allergic rhinitis, and atopic dermatitis as an anti-allergy drug (JP-A-2000-95697) are known.

As skin disorder drugs for accelerating skin wound healing by utilizing lactic acid bacteria as a natural substance, a mixture of a cell-activating agent such as lactic acid bacteria and alkaline simple hot spring water, water soluble rutin, an asparagus extract, or an extracts from plants selected from dried flower bud of Rosa rugosa, dried seed of Prunus japonica, and dried fruit of Chaenomeles lagenaria are disclosed in JP-A 10-182411, JP-A-08-099860, JP-A- 06-128140 and JP-A-06-065041

JP 11276072A discloses a fermented material obtained by subtaining a lactic acid bacterium capable of proliferating in green tea useful as a healthfood, JP2005013211A discloses a healthfood including Lactobacillii and a green tea extract; and US 6383482B1 describes a weight loss composition containing Lactobacillii and green tea.

The present inventor has aimed at green tea for substituting the above-mentioned natural substances and has found the fact that cultures prepared by culturing bacteria in the presence of green tea are highly safe and that topical drugs (ointments), skin disorder drugs, cosmetics, and drugs such as orally administered drugs that have high effects of suppressing dermatitis development and accelerating skin wound healing, and quasi-drugs for prevention or improvement can be obtained by using the bacterial cultures.

It is an object of the present invention to provide cultures prepared by culturing bacteria in the presence of green tea and products using these cultures, such as topical drugs (ointments), skin disorder drugs, cosmetics, drugs such as orally administered drugs, and quasi-drugs for prevention or improvement, which have high effects of suppressing dermatitis development and accelerating skin wound healing, and are natural substances with high safety and also high versatility.

Kefir, of which the birthplace is thought to be Caucasus, is a traditional composite fermented milk of lactic acid bacteria and yeast. Kefir is prepared by culturing kefir grains serving as a starter in milk.

The inventor has found that this natural substance, kefir grains, is safe for human and animals even if it is used as a drink, a food, a cosmetic, or a feed, and has further conducted intensive studies on various usages of kefir grains. The inventor has found that a culture liquid that is prepared by culturing kefir grains in a green tea extract and then removing bacterial cells from the bacterial culture has an excellent skin-bleaching effect. The inventor has further found that the kefir grains have a high anti-oxidizing ability and has filed a patent application as a cosmetic (JP-A-2004-149442).

A patent application has been already filed for a bacterial culture obtained by culturing bacteria separated from kefir grains or a bacteria group containing the bacteria, and products prepared by using them (Japanese Patent Application No. 2004-175969, Application date: June 14, 2004); EP 1 607 476 A1 which is a document according to A 54 (3) EPC. Furthermore, a patent application has been already filed for a bacterial culture obtained by culturing bacteria separated from kefir grains or a bacteria group containing the bacteria in a culture medium containing a mugwort extract liquid as a main ingredient, and products prepared by using them (Japanese Patent Application No. 2004-311983, Application date: October 27, 2004).

In addition, a patent application has been already filed for a bacterial culture obtained by culturing bacteria separated from kefir grains or a bacteria group containing the bacteria in a culture medium containing green tea and black tea as main ingredients, and products prepared by using them (Japanese Patent Application No. 2005-091618, Application date: March 28, 2005). Similarly, a patent application has been already filed for a bacterial culture obtained by culturing bacteria separated from kefir grains or a bacteria group containing the bacteria in a culture medium containing green tea, black tea, and a mugwort extract liquid as main ingredients, and products prepared by using them (Japanese Patent Application No. 2005-091624, Application date: March 28, 2005).

Furthermore, as a result of further studies, the inventor has found the fact that cultures prepared by culturing bacteria separated from kefir grains, or a bacteria group containing these bacteria, in the presence of green tea or a combination of green tea and another tea other than green tea and/or an effective ingredient thereof have effects of suppressing dermatitis development and accelerating skin wound healing in human or animals and the cultures can be used as raw materials of topical drugs (ointments), skin disorder drugs, cosmetics, drugs such as orally administered drugs, and quasi-drugs for prevention or improvement or can be used by being added to these products or their raw materials. Then, the inventor has carried out the development of products having effects of suppressing dermatitis development and accelerating skin wound healing by adding the cultures to topical drugs (ointments), skin disorder drugs, cosmetics, drugs such as orally administered drugs, and quasi-drugs for prevention or improvement.

### [Means for Solving the Problems]

The present invention is put into practice by the following embodiments, namely, the invention relates to
(1) A bacterial culture that has an effect of suppressing dermatitis development and is prepared by culturing new bacteria separated from kefir grains and belonging to the family Lactobacillaceae, or a bacteria group containing the new bacteria, in the presence of green tea;
(2) A bacterial culture that has an effect of accelerating skin wound healing and is prepared by culturing new bacteria separated from kefir grains and belonging to the family Lactobacillaceae or a bacteria group containing the new bacteria in the presence of green tea ;
(3) A topical drug (ointment) and a quasi-drug for prevention or improvement that have effects of suppressing dermatitis development and accelerating skin wound healing and contain a bacterial culture prepared by culturing new bacteria separated from kefir grains and belonging to the family Lactobacillaceae or a bacteria group containing the new bacteria in the presence of green tea ;
(4) A skin disorder drug and a quasi-drug for prevention or improvement that have effects of suppressing dermatitis development and accelerating skin wound healing and contain a bacterial culture prepared by culturing new bacteria separated from kefir grains and belonging to the family Lactobacillaceae or a bacteria group containing the new bacteria in the presence of green tea;
(5) A cosmetic that has an effect of suppressing dermatitis development and contains a bacterial culture prepared by culturing new bacteria separated from kefir grains and belonging to the family Lactobacillaceae or a bacteria group containing the new bacteria in the presence of green tea ; and
(6) An orally administered drug and a quasi-drug for prevention or improvement that have an effect of suppressing dermatitis development and contain a bacterial culture prepared by culturing new bacteria separated from kefir grains and belonging to the family Lactobacillaceae or a bacteria group containing the new bacteria in the presence of green tea.
(7) In addition, a culture prepared by removing bacterial cells from the culture in any one of the above-described (1) to (6) has an effect and a function similar to those in above, namely, effects of suppressing dermatitis development and/or accelerating skin wound healing.

Here, the term "effect of suppressing dermatitis development" means the effect that skin inflammation, an allergic symptom, or itching is suppressed when a topical drug (ointment), a skin disorder drug, a cosmetic, a drug such as an orally administered drug, or a quasi-drug for inhibition or improvement is administered to human or an animal. The term "effect of accelerating skin wound healing" means the effect of promoting improvement of recovery so that the time spent on the recovery when a topical drug (ointment), a skin disorder drug, a cosmetic, a drug such as an orally administered drug, or a quasi-drug for inhibition or improvement is administered to human or an animal is shortened to less than that when the human or the animal left without treatment and cured by recuperative powers and natural healing powers of living bodies. The term "bacterial culture" includes a culture obtained by culturing a bacteria group separated from kefir grains or singular bacteria separated from kefir grains and a culture obtained by removing bacterial cells from the culture.

### [Advantages of the Invention]

According to embodiment 1 of the invention, a bacterial culture prepared by culturing new bacteria separated from kefir grains and belonging to the family Lactobacillaceae or a bacteria group containing the new bacteria in the presence of green tea and having an effect of suppressing dermatitis development can be newly provided. The culture is safe without toxicity or side effects and can be applied to various fields as topical drugs (ointments), skin disorder drugs, cosmetics, drugs such as orally administered drugs, and quasi-drugs for prevention or improvement.

According to embodiment 2 of the invention, a bacterial culture prepared by culturing new bacteria separated from kefir grains and belonging to the family Lactobacillaceae or a bacteria group containing the new bacteria in the presence of green tea and having an effect of accelerating skin wound healing can be newly provided. The culture is safe without toxicity or side effects and can be applied to various fields as drugs such as topical drugs (ointments) and skin disorder drugs and quasi-drugs for prevention or improvement.

According to embodiments 3 and 4 of the invention, a drug such as a topical drug (ointment) and a skin disorder drug and a quasi-drug for prevention or improvement that have effects of suppressing dermatitis development and accelerating skin wound healing and contain a bacterial culture prepared by culturing new bacteria separated from kefir grains and belonging to the family Lactobacillaceae or a bacteria group containing the new bacteria in the presence of green tea can be newly provided.

According to embodiments 5 and 6 of the invention, a cosmetic, a drug such as an orally administered drug, and a quasi-drug for prevention or improvement that have an effect of suppressing dermatitis development and contain a bacterial culture prepared by culturing new bacteria separated from kefir grains and belonging to the family Lactobacillaceae or a bacteria group containing the new bacteria in the presence of green tea can be newly provided.

According to embodiment 7 of the invention, a culture that has an effect and a function of suppressing dermatitis development and/or accelerating skin wound healing and is prepared by removing bacterial cells from the culture in any one of the embodiments 1 to 6 can be newly provided.

### [Brief Description of the Drawings]

Figure 1 is a graph showing the relationship between curing term and body weight in a dermatitis development suppression test.
Figure 2 is a graph showing the relationship between curing term and dermatitis score in a dermatitis development suppression test.
Figure 3 is a graph showing the relationship between curing term and dermatitis occurrence rate in a dermatitis development suppression test.
Figure 4 is a graph showing comparison of tensile strength in a skin wound healing acceleration evaluation test.

### [Best Mode for Carrying Out the Invention]

Examples of the present invention will be described in detail.
A bacteria group separated from kefir grains includes Acetobacter cerevisiae strain SIID1719-2b, Gluconobacter oxydans strain SIID1719-3b, a new species Lactobacillus sp. SIID1719-6b of the family Lactobacillaceae, Pichiamembrani faciens strain SIID1719-1y, Saccharomyces cerevisiae strain SIID1719-4y, and Pichia anomala strain SIID1719-5y.

The bacteria group separated from kefir grains and the new species Lactobacillus sp. SIID1719-6b of the family Lactobacillaceae were each cultured in the presence of green tea, and bacterial cells were removed from the resulting bacterial cultures. The thus obtained cultures were subjected to the following evaluation tests whether the cultures have effects of suppressing dermatitis development and accelerating skin wound healing. The results confirmed that the bacteria group and Lactobacillus sp. SIID1719-6b alone each had the effects of suppressing dermatitis development and accelerating skin wound healing. Furthermore, in the present invention, cultures having a similar function, which are prepared by culturing Lactobacillus sp. SIID1719-6b together with symbiotic bacteria the safety of which has been already confirmed, can be used. In addition, the culture medium may contain tea other than green tea and/or an effective ingredient thereof, in addition to green tea. The new species Lactobacillus sp. SIID1719-6b of the family Lactobacillaceae has been deposited at International Patent Organism Depositary, the National Institute of Advanced Industrial Science and Technology, Japan as FERM AP-20062, and transferred to the international deposition as FERM ABP-10299.

As a typical example, a culture prepared by culturing the new species Lactobacillus sp. SIID1719-6b of the family Lactobacillaceae in a culture medium containing green tea and then removing the bacterial cells from the resulting bacterial culture was used. Evaluation tests for effects of suppressing dermatitis development and accelerating skin wound healing were performed as follows:

### (Dermatitis development suppression test)

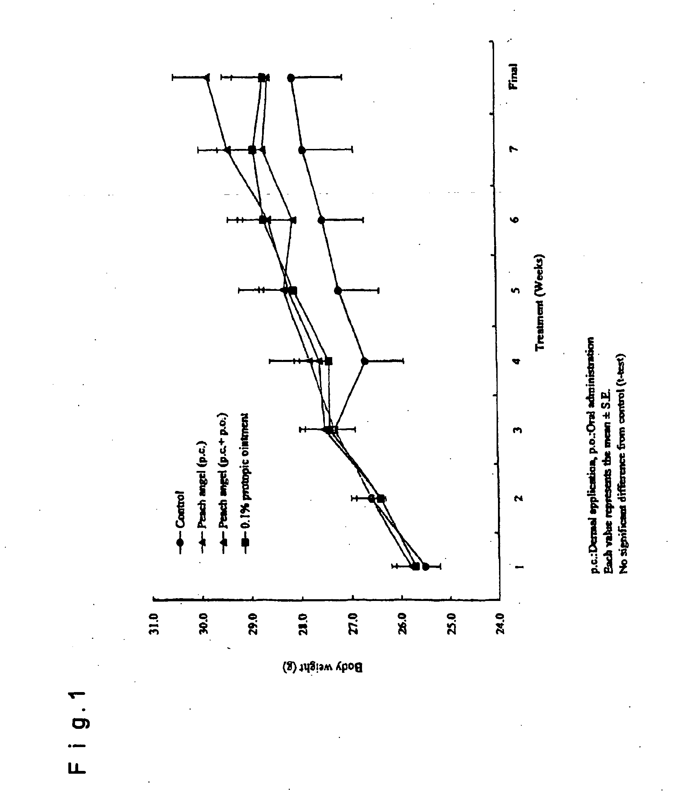

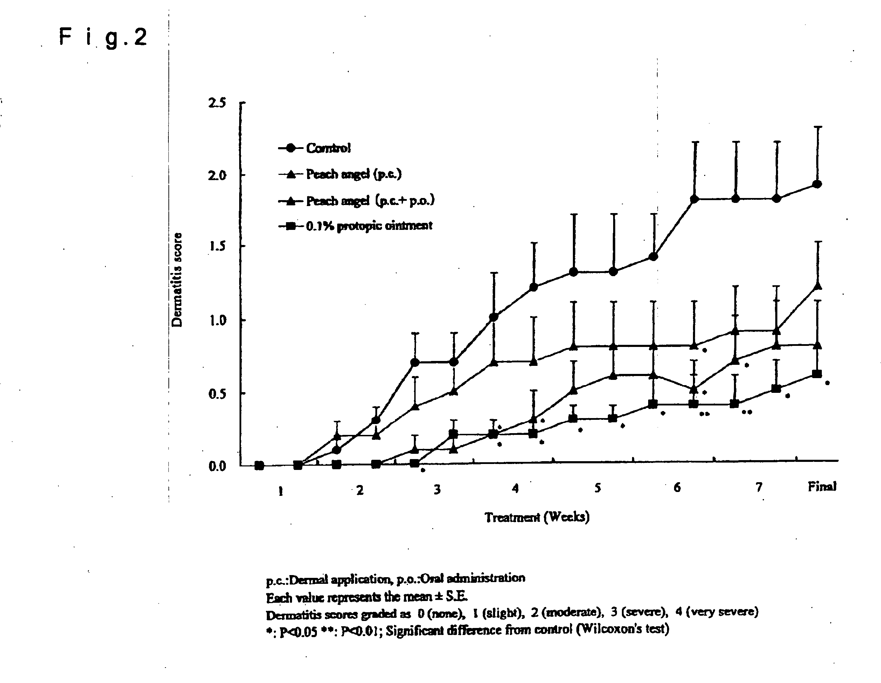

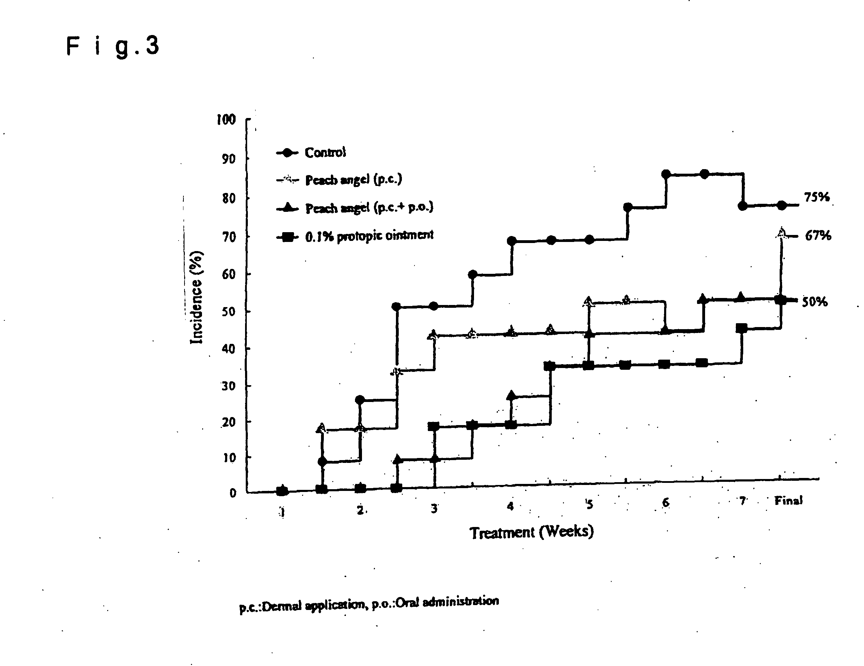

First, NC/Nga mice, which spontaneously develop dermatitis at a high frequency under usual feeding conditions without conducting air cleaning, were assigned to a non-treatment group as a control, a peach angel (dermal application) group, a peach angel (dermal application and oral administration) group, and a 0.1% Protopic ointment (dermal application) group as a comparative group according to the body weights using the stratified sequence randomization technique (average weight in each group: 25.5 to 25.8 kg). Cultures for the peach angel groups were prepared by culturing SIID1719-6b in a culture medium containing green tea for 48 hours and then removing the bacterial cells from the resulting bacterial culture. Each group consists of 12 mice. The feeding conditions, i.e., temperature, humidity, feed, drink, and feeding environment, were the same for all mice. Mice of the peach angel (dermal application) group were transdermally administered with 0.10 mL of the culture, mice of the peach angel (dermal application and oral administration) group were administered transdermally with 0.10 mL and orally with 10 mL/kg body weight of the culture, and mice of the 0.1 % Protopic ointment (dermal application) group were transdermally administered with 0.10 g of 0.1 % Protopic ointment. The administration was performed to 6-week old mice once a day for 49 times (7 weeks). The weight of each mouse was measured once a week and the conditions of skin were observed twice a week according to the dermatitis evaluation standard (dermatitis score: 5-level evaluation, see Table 1). The resulting changes in the body weight are shown in Figure 1, dermatitis scores are shown in Figure 2, and rates of dermatitis occurrence are shown in Figure 3.

**[Table 1]**

| Dermatitis Observation Score | |
|---|---|
| Score | Symptom |
| 0 (Normal) | No occurrence of dermatitis or complete cure |
| 1 (Light) | Fairly light erythema, light wound, or similar degree of cure |
| 2 (Moderate) | Obvious erythema, wound, light bleeding, or similar degree of cure |
| 3 (Severe) | Moderate to severe erythema, wound (partial defluxion of auricle), localized ulcer, incrustation, or similar degree of cure |
| 4 (Defluxion of auricle) | A large amount of bleeding, defluxion of auricle with bleeding, or a wide area of ulcer |

The body weight of the non-treatment group decreased in the fourth week, but slightly increased afterward. However, the body weight on the last day was the lightest among the tested groups. No significant difference was observed in the 0.1 % Protopic ointment group and the peach angel (dermal application) group, and a decrease in the body weight on the last day was confirmed in both groups.
The body weight of the peach angel (dermal application and oral administration) group increased from the start to the end of the administration, and the body weigh on the last day was the highest.

With respect to the occurrence degree of dermatitis, the non-treatment group had an average score of 1.9 on the last day and an occurrence rate of 75%, which is the highest dermatitis score. The 0.1% Protopic ointment group had an average score of 0.6 on the last day and an occurrence rate of 50%, which is the lowest dermatitis score. A significant difference compared with the non-treatment group was confirmed in the effect of suppressing dermatitis development.

The peach angel (dermal application) group had an average score of 1.2 on the last day and an occurrence rate of 67%, and the peach angel (dermal application and oral administration) group had an average score of 0.8 on the last day and an occurrence rate of 50%. These groups have suppressing effects lower than that of the 0.1 % Protopic ointment group, but a significant difference compared with the non-treatment group was confirmed in the effect of suppressing the development. In addition, it was confirmed that the effect of suppressing the development in the peach angel (dermal application and oral administration) group was higher than that in the peach angel (dermal application) group. This suggests that a more effective function of suppressing the development is achieved by oral administration. Furthermore, the safety was confirmed again.

### (Skin wound healing acceleration evaluation test)

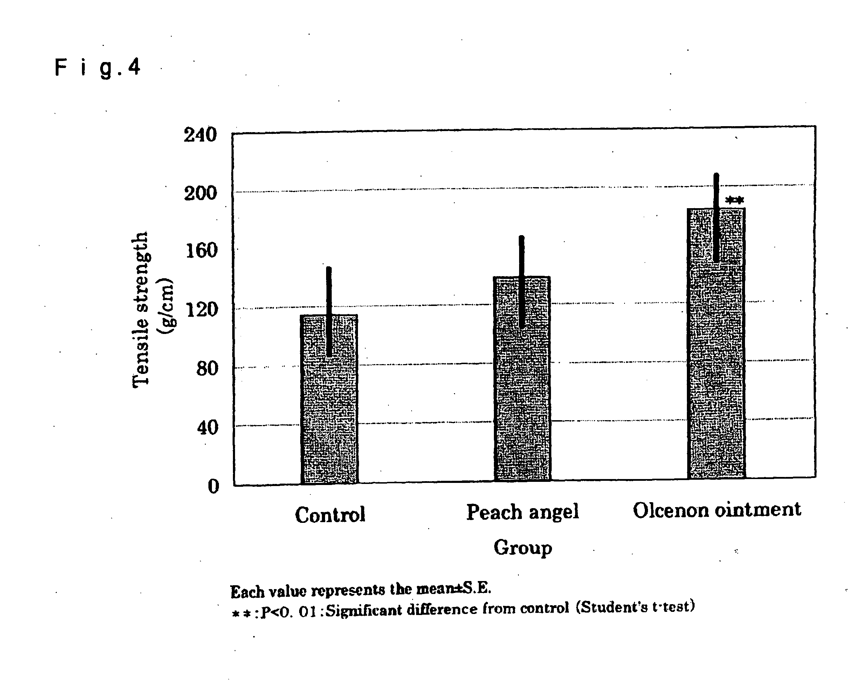

First, the skin of each male Slc:Wistar rat was incised approximately about 2.5 cm under sodium pentobarbital anesthesia and was then sutured at a part of the central portion. The suture was removed after three days. The rats were assigned to a non-treatment group as a control, a peach angel group to be treated with a culture prepared by culturing SIID1719-6b in a culture medium containing green tea for 48 hours and then removing the bacterial cells from the bacterial culture, and an Olcenon ointment group as a comparative group according to the body weights so that the average body weight of each group is approximately the same. Each group consists of 8 rats. The feeding conditions, i.e., temperature, humidity, feed, drink, and feeding environment, were the same for all rats. Rats of the peach angel group were administered with 0.10 mL of the culture for each site once a day for 12 days from the date of the grouping (the first day). Rats of the Olcenon ointment group were administered with 100 mg of Olcenon ointment for each site once a day for 6 days from the date of skin incision (the seventh day). On the next day (the thirteenth day) of the last administration of the tested substances, the effect of accelerating healing was investigated by measuring the tensile strength (g/cm) necessary for recleavage of the wound site with a push-pull gauge. Figure 4 shows the results.

The term tensile strength means the force necessary for cleaving the wound site where was incised and cured. The higher value means a larger force necessary for recleavage, which reflects accelerated healing. It was confirmed from the measurement results that the tensile strength of the beach angel group was lower than that of the Olcenon ointment group, but higher than that of the non-treatment group. Therefore, it is suggested that the peach angel group has an effect of accelerating skin wound healing.

In the above-described embodiment, a culture prepared by culturing the new species Lactobacillus sp. SIID1719-6b of the family Lactobacillaceae separated from kefir grains in a culture medium containing green tea and then removing the bacterial cells from the resulting bacterial culture was used. Cultures prepared by culturing the new bacteria separated from kefir grains and belonging to the family Lactobacillaceae or a bacteria group containing the new bacteria in the presence of green tea or a combination of green tea and another tea other than green tea and/or an effective ingredient thereof are expected to have effects equivalent to those exemplarily shown here, namely, effects of suppressing dermatitis development and accelerating skin wound healing.

Furthermore, drugs such as topical drugs (ointments) and skin disorder drugs and quasi-drugs for prevention or improvement which contain these cultures can be expected to have effects of suppressing dermatitis development and accelerating skin wound healing. In addition, cosmetics containing these cultures can be expected to have an effect of suppressing dermatitis development. Furthermore, drugs such as orally administered drugs and quasi-drugs for prevention or improvement containing these cultures can be expected to have an effect of suppressing dermatitis development.

## Claims

1. A composition for use as a medicament, the composition being ***characterized by*** comprising a bacterial culture which is prepared by culturing new bacteria separated from kefir grains, belonging to the family Lactobacillaceae and having the international deposition no. FERM ABP-10299 in the presence of green tea or by comprising a bacterial culture product which is prepared by removing bacterial cells from said bacterial culture.

2. A composition for use in the treatment of dermatitis, the composing being ***characterized by*** comprising a bacterial culture which is prepared by culturing new bacteria separated from kefir grains, belonging to the family Lactobacillaceae and having the international deposition no. FERM ABP-10299 in the presence of green tea or by comprising a bacterial culture product which is prepared by removing bacterial cells from said bacterial culture.

3. A composition for use in the treatment of skin wounds, the composition being ***characterized by*** comprising a bacterial culture which is prepared by culturing new bacteria separated from kefir grains, belonging to the family Lactobacillaceae and having the international deposition no. FERM ABP-10299 in the presence of green tea or by comprising a bacterial culture product which is prepared by removing bacterial cells from said bacterial culture.

4. The composition as claimed in any one of claims 1 to 3, wherein the composition is for topical use.

5. The composition as claimed in any one of claims 1 to 3, wherein the composition is for oral administration.

6. The composition as claimed in any one of claims 1 to 3, wherein the composition is for dermal application and oral administration.

## Patentansprüche

1. Zusammensetzung zur Verwendung als Medikament, wobei die Zusammensetzung **dadurch gekennzeichnet ist, dass** sie eine bakterielle Kultur umfasst, die hergestellt wird durch Kultivieren neuer Bakterien, die aus Kefirkörnern abgetrennt wurden, die zur Familie Lactobacillaceae gehören und die internationale Hinterlegungsnummer FERM ABP-10299 aufweisen, in der Gegenwart von grünem Tee oder dass sie ein bakterielles Kulturprodukt umfassen, das hergestellt wird durch Entfernen von bakteriellen Zellen aus der bakteriellen Kultur.

2. Zusammensetzung zur Verwendung bei der Behandlung von Dermatitis, wobei die Zusammensetzung **dadurch gekennzeichnet ist, dass** sie eine bakterielle Kultur umfasst, die hergestellt wird durch Kultivieren neuer Bakterien, die aus Kefirkörnern abgetrennt wurden, die zur Familie Lactobacillaceae gehören und die internationale Hinterlegungsnummer FERM ABP-10299 aufweisen, in der Gegenwart von grünem Tee oder dass sie ein bakterielles Kulturprodukt umfassen, das hergestellt wird durch Entfernen von bakteriellen Zellen aus der bakteriellen Kultur.

3. Zusammensetzung zur Verwendung bei der Behandlung von Hautwunden, wobei die Zusammensetzung **dadurch gekennzeichnet ist, dass** sie eine bakterielle Kultur umfasst, die hergestellt wird durch Kultivieren neuer Bakterien, die aus Kefirkörnern abgetrennt wurden, die zur Familie Lactobacillaceae gehören und die internationale Hinterlegungsnummer FERM ABP-10299 aufweisen, in der Gegenwart von grünem Tee oder dass sie ein bakterielles Kulturprodukt umfassen, das hergestellt wird durch Entfernen von bakteriellen Zellen aus der bakteriellen Kultur.

4. Zusammensetzung gemäß einem der Ansprüche 1 bis 3, wobei die Zusammensetzung zur topischen Verwendung dient.

5. Zusammensetzung gemäß einem der Ansprüche 1 bis 3, wobei die Zusammensetzung zur oralen Verabreichung dient.

6. Zusammensetzung gemäß einem der Ansprüche 1 bis 3, wobei die Zusammensetzung zur dermalen Anwendung und oralen Verabreichung dient.

## Revendications

1. Composition utilisable comme médicament, la composition étant **caractérisée en ce qu'**elle comprend une culture bactérienne qui est préparée en cultivant de nouvelles bactéries séparées de grains de kéfir, appartenant à la famille des *Lactobacillaceae* et ayant le numéro de dépôt international FERM ABP-10299, en présence de thé vert ou **en ce qu'**elle comprend un produit de culture bactérienne qui est préparé en retirant les cellules bactériennes de ladite culture bactérienne.

2. Composition utilisable dans le traitement d'une dermatite, la composition étant **caractérisée en ce qu'**elle comprend une culture bactérienne qui est préparée en cultivant de nouvelles bactéries séparées de grains de kéfir, appartenant à la famille des *Lactobacillaceae* et ayant le numéro de dépôt international FERM ABP-10299, en présence de thé vert ou **en ce qu'**elle comprend un produit de culture bactérienne qui est préparé en retirant les cellules bactériennes de ladite culture bactérienne.

3. Composition utilisable dans le traitement des blessures cutanées, la composition étant **caractérisée en ce qu'**elle comprend une culture bactérienne qui est préparée en cultivant de nouvelles bactéries séparées de grains de kéfir, appartenant à la famille des *Lactobacillaceae* et ayant le numéro de dépôt international FERM ABP-10299, en présence de thé vert ou **en ce qu'**elle comprend un produit de culture bactérienne qui est préparé en retirant les cellules bactériennes de ladite culture bactérienne.

4. Composition selon l'une quelconque des revendications 1 à 3, dans laquelle la composition est destinée à un usage topique.

5. Composition selon l'une quelconque des revendications 1 à 3, dans laquelle la composition est destinée à une administration par voie orale.

6. Composition selon l'une quelconque des revendications 1 à 3, dans laquelle la composition est destinée à une application cutanée et à une administration par voie orale.
